(19) Europäisches Patentamt
European Patent Office
Office européen des brevets

(11) Veröffentlichungsnummer: **0 391 393 A2**

(12) # EUROPÄISCHE PATENTANMELDUNG

(21) Anmeldenummer: **90106443.6**

(51) Int. Cl.$^5$: **C07D 501/36**

(22) Anmeldetag: **04.04.90**

(30) Priorität: **07.04.89 DE 3911322**

(43) Veröffentlichungstag der Anmeldung: **10.10.90 Patentblatt 90/41**

(84) Benannte Vertragsstaaten: **AT BE CH DE DK ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Martin, Wolfgang, Dr.**
**Am Schieferberg 19**
**D-6233 Kelkheim/Taunus(DE)**

(54) **Verfahren zur Herstellung von Cefodizim-Dinatrium.**

(57) Verfahren zur Herstellung von Cefodizim-Dinatrium, dadurch gekennzeichnet, daß man Cefodizim mittels 1,5 - 2,5 Mol einer organischen Aminbase in Ethanol mit einem Wassergehalt von 4 - 15 % in Lösung bringt und das Dinatriumsalz des Cefodizims durch Zugabe eines Natriumspenders auskristallisieren läßt.

EP 0 391 393 A2

## Verfahren zur Herstellung von Cefodizim-Dinatrium

Nach der EP-PS 78 532 wird kristallines Cefodizim-Dinatrium durch Lösen von Cefodizim in Wasser mittels Natriumbicarbonat und anschließende Kristallisation durch Ethanol- oder Isopropanolzugabe hergestellt.

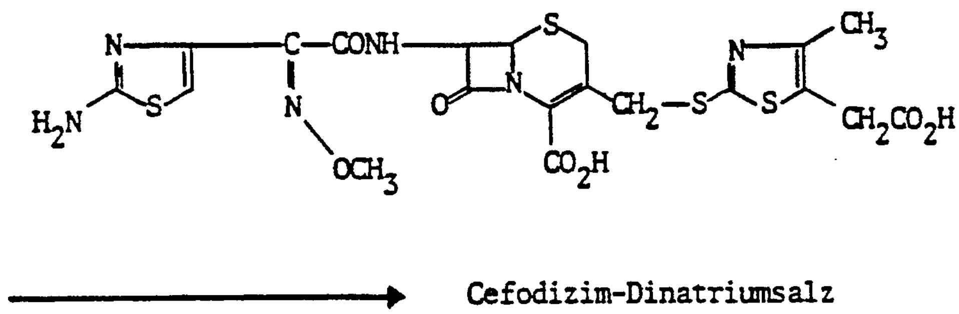


Unter Praxisbedingungen wird ein Wasser-Ethanol (2:1)-Gemisch angewandt (3 Teile), unter Zugabe 10 %iger wäßriger Natronlauge (1,2 Teile), so daß der Wassergehalt der anfangs hergestellten Cefodizim-Dinatrium-Lösung etwa 50 % beträgt. Nach Sterilfiltration wird Cefodizim-Dinatrium mit etwa 20 Teilen Ethanol ausgefällt. Aufgrund des Wasseranteils von etwa 50 % in der Lösung treten entsprechende Verluste allein aufgrund der Löslichkeit des Produktes in Wasser auf. Der Wassergehalt erfordert auch eine entsprechend große Fällethanolmenge, woraus sich auch eine schlechte Volumennutzung ergibt. Außerdem muß zeitweilig mit einer übersättigten Lösung gearbeitet werden, mit der Gefahr einer unkontrollierten Auskristallisation. Weiterhin werden zum Lösen mittels 2,5 n Natronlauge mindestens 1 - 2 Stunden benötigt. Eine gewisse Produktschädigung durch lokale Überalkalisierung durch Natronlauge und durch die lange Lösezeit ist bei diesem Verfahren nicht auszuschließen.

Es wurde nun gefunden, daß man Cefodizim-Dinatrium wesentlich leichter, schonender, ökonomischer und mit wesentlich höherer Ausbeute (über 85 % der Theorie, statt etwa 60 %) herstellen kann, indem man Cefodizim-Säure in Ethanol, wobei das Ethanol einen Wassergehalt von etwa 4 - 15 %, vorzugsweise von 5 - 6 %, besitzen muß, durch Zugabe von 1,5 - 2,5 Mol, vorzugsweise 1,8 - 2,2 Mol, insbesondere 2 Mol einer organischen Aminbase, vorzugsweise von Triethylamin auf schonende Weise und, was für eine rationelle Produktion von Bedeutung ist, praktisch momentan in Lösung bringt und das Dinatriumsalz des Cefodizims durch Zugabe eines Natriumspenders auskristallisieren läßt.

Als Natriumspender kommt vorzugsweise Natrium-2-ethylhexanoat in Betracht.

Zweckmäßigerweise wird der Lösevorgang unter leichtem Kühlen der Reaktionslösung auf etwa 5 - 10 °C durchgeführt. Unterhalb 0 °C ist die Auflösung erschwert.

Überraschenderweise sind für diesen Lösevorgang geringe Mengen Wasser (etwa 4 - 15 %, vorzugsweise etwa 5 - 6 % der Gesamtmischung) ganz wesentlich. Ohne diesen Wasserzusatz erholt man beim Versuch, Cefodizimsäure in den mit Wasser mischbaren Alkoholen Ethanol, Isopropanol und n-Propanol oder in Aceton zu lösen, dicke, nicht filtrierbare Gallerten. Erst der geringe Wasserzusatz bewirkt die glatte Auflösung.

Bei der bevorzugten Ausführungsform des erfindungsgemäßen Verfahrens mit Ethanol erhält man unter Zuhilfenahme vergleichsweise geringer Wassermengen (Anteil der Gesamtmischung vorzugsweise etwa 5 - 6 %) eine überwiegend ethanolische Lösung, die problemlos sterilfiltriert werden kann, ohne daß vorzeitige Auskristallisation des Natriumsalzes, besonders unter Betriebsbedingungen, befürchtet werden muß.

Aus der gegebenenfalls mit Kohle geklärten und (steril-) filtrierten Lösung wird Cefodizim-Dinatrium durch Zugabe von 2 Moläquivalenten eines geeigneten Natriumspenders, vorzugsweise von Natrium-2-ethylhexanoat, ebenfalls gelöst in Ethanol, kristallisiert. Zweckmäßigerweise wird ein etwa 10 - 15 %iger Überschuß angewandt. Nach Zugabe von etwa einem Drittel der theoretisch benötigten Reagenzmenge beginnt die Abscheidung des Cefodizim-Dinatriumsalzes in kristalliner Form. Sie kann durch Animpfen gefördert werden. Die Kristallisation wird bei etwa 5 - 20 °C durchgeführt. Die Temperatur steigt während der Natrium-2-ethylhexanoat-Zugabe von etwa 10 auf etwa 20 °C an. Die Kristallsuspension wird noch etwa 1 Stunde bei Raumtemperatur gerührt, die Kristalle abgesaugt, mit Ethanol gewaschen und im Vakuum bei 40 °C getrocknet.

Die wesentlichen Vorteile des Verfahrens liegen

- in der höheren Ausbeute (über 85 % d.Th., gegenüber etwa 60 % nach dem bisherigen Verfahren),
- in der erheblich höheren (doppelten) Volumennutzung,
- in dem wesentlich vereinfachten und zeitlich stark verkürzten Lösen, und damit dem Ausbleiben einer möglichen Produktschädigung,
- in dem Ausbleiben einer unkontrollierten Auskristallisation, sowie
- in etwas günstigeren Produkteigenschaften, wie dem Fehlen einer Tendenz zu elektrostatischer Aufladung.

Es war nicht vorauszusehen, daß das sehr einfache erfindungsgemäße Verfahren zu den vorstehend genannten Vorteilen führen würde.

**Beispiel 1**

35,04 g Cefodizim-Säure (95 %ig; Wassergehalt 2,1 %) werden in 250 ml Ethanol mit 18 ml Triethylamin unter Stickstoffüberlagerung während 5 Minuten bei 7 - 10° C unter Kühlen versetzt. Danach werden unter guter Kühlung 15 ml Wasser bei 7 - 10° C während 1 - 2 Minuten zugetropft.

Man gibt 1,0 g SX II-Kohle zu, rührt 10 Minuten bei etwa 5° C, filtriert und tropft bei etwa 10° C bis Raumtemperatur (etwa 20° C) eine filtrierte Lösung von 20,3 g Natrium-2-ethylhexanoat und 2 ml 2-Ethylhexansäure in 90 ml Ethanol während 1 (-2) Stunden zu.

Nach Zugabe von 20 - 30 ml der Natrium-2-ethyl-hexanoat-Lösung wird wiederholt angeimpft. Nach der Natrium-2-ethylhexanoat-Zugabe wird die resultierende Suspension noch 1,5 - 2 Stunden bei Raumtemperatur (etwa 20° C) gerührt. Man saugt ab, wäscht mit 200 ml Ethanol und trocknet, zuerst bei Raumtemperatur, dann bei 45° C im Vakuum bis zur Gewichtskonstanz.
Ausbeute: 32,3 g (85 % d.Th.)

**Ansprüche**

1. Verfahren zur Herstellung von Cefodizim-Dinatrium, dadurch gekennzeichnet, daß man Cefodizim mittels 1,5 - 2,5 Mol einer organischen Aminbase in Ethanol mit einem Wassergehalt von 4 - 15 % in Lösung bringt und das Dinatriumsalz des Cefodizims durch Zugabe eines Natriumspenders auskristallisieren läßt.

2. Verfahren nach Anspruch 1, dadurch gekennzeichnet, daß der Wassergehalt des Ethanols 5 - 6 % beträgt.

3. Verfahren nach Ansprüchen 1 bis 2, dadurch gekennzeichnet, daß als organische Aminbase Triethylamin eingesetzt wird.

4. Verfahren nach Ansprüchen 1 bis 3, dadurch gekennzeichnet, daß der Natriumspender das Natrium-2ethylhexanoat ist.

5. Verfahren nach Ansprüchen 1 bis 4, dadurch gekennzeichnet, daß der Natriumspender in einem Überschuß von 10 - 15 % eingesetzt wird.